# EUROPEAN PATENT APPLICATION

(11) **EP 4 124 286 A1**
(43) Date of publication of application: **01.02.2023**
(21) Application number: 22186225.3
(22) Date of filing: 21.07.2022
(51) Int. Cl.: A61B 5/00

(54) **METHOD FOR MANUFACTURING PHYSIOLOGICAL INFORMATION ACQUISITION APPARATUS AND PHYSIOLOGICAL INFORMATION ACQUISITION APPARATUS**

(30) Priority: 30.07.2021 JP 2021125500
(71) Applicant: Nihon Kohden Corporation, Shinjuku-ku Tokyo (JP)
(72) Inventor: Masamura, Mikio, Tokorozawa-shi, Saitama (JP)
(74) Representative: Grünecker Patent- und Rechtsanwälte PartG mbB

(57) **Abstract**

A method for manufacturing a physiological information acquisition apparatus configured to acquire physiological information of a subject includes: preparing a common first case that defines an internal space in which a processing device configured to perform processing of acquiring the physiological information is accommodated; preparing a plurality of types of display devices including different sizes of screens for displaying an image including the physiological information; preparing a plurality of types of second cases each for supporting a corresponding type of display device among the plurality of types of display devices; selecting one type of display device from the plurality of types of display devices; selecting, from the plurality of types of second cases, one type of second case corresponding to the selected one type of display device; and coupling the first case to a coupling portion provided on a back wall of the second case. The back wall faces the internal space.

## Description

### TECHNICAL FIELD

The presently disclosed subject matter relates to a physiological information acquisition apparatus for acquiring physiological information of a subject. The presently disclosed subject matter also relates to a method for manufacturing the physiological information acquisition apparatus.

### BACKGROUND ART

International Publication No. WO 2020/087453 (hereinafter, referred to as PTL 1) discloses a physiological information acquisition apparatus for acquiring physiological information of a subject. The physiological information acquisition apparatus includes a first case and a second case which are coupled. The first case accommodates a processing device for acquiring physiological information. The second case supports a display device for displaying an image including the physiological information.

There may be a plurality of types of the above-described physiological information acquisition devices including display devices of different sizes. Therefore, dimensions of the second case for supporting the display device and dimensions of the first case coupled to the second case are also different among the plurality of types.

An object of the presently disclosed subject matter is to prevent an increase in the number of necessary dies caused by an increase in the number of types of a physiological information acquisition apparatus.

### SUMMARY

A first aspect of the presently disclosed subject matter relates to a method for manufacturing a physiological information acquisition apparatus configured to acquire physiological information of a subject. The method includes: preparing a common first case that defines an internal space in which a processing device configured to perform processing of acquiring the physiological information is accommodated; preparing a plurality of types of display devices including different sizes of screens for displaying an image including the physiological information; preparing a plurality of types of second cases each for supporting a corresponding type of display device among the plurality of types of display devices; selecting one type of display device from the plurality of types of display devices; selecting, from the plurality of types of second cases, one type of second case corresponding to the selected one type of display device; and coupling the first case to a coupling portion provided on a back wall of the second case. The back wall faces the internal space.

According to the configuration described above, even when it is necessary to prepare a plurality of types of second cases in order to support the display devices having different screen sizes according to diversified needs, it is not necessary to prepare a plurality of types of first cases having different specifications so as to respectively correspond to the plurality of types of second cases. It is significant to eliminate the necessity of preparing a plurality of types of dies in order to manufacture the first cases, for example, for efficiency. Therefore, an increase in the number of necessary dies caused by an increase in the number of types of the physiological information acquisition apparatus can be prevented.

A second aspect of the presently disclosed subject matter relates to a physiological information acquisition apparatus configured to acquire physiological information of a subject. The physiological information acquisition apparatus includes: a processing device configured to perform processing of acquiring the physiological information; a display device configured to display an image including the physiological information; a first case that defines an internal space for accommodating the processing device; and a second case configured to support the display device. The second case include a back wall facing the internal space, and a coupling portion provided on the back wall and coupled to the first case.

### BRIEF DESCRIPTION OF DRAWINGS

FIG. 1 illustrates an appearance of a vital monitor according to an embodiment.
FIG. 2 illustrates an appearance of the vital monitor according to the embodiment.
FIG. 3 illustrates a cross section taken along a line III-III in FIG. 1 as viewed in a direction of arrows.
FIG. 4 illustrates an appearance of a first case according to the embodiment.
FIG. 5 illustrates a method for manufacturing the vital monitor according to the embodiment.
FIG. 6 illustrates the method for manufacturing the vital monitor according to the embodiment.
FIG. 7 illustrates an example of an area VII in FIG. 3 in an enlarged manner.
FIG. 8 illustrates another example of an area VII in FIG. 3 in an enlarged manner.

### DESCRIPTION OF EMBODIMENTS

Exemplary embodiments will be described in detail below with reference to the accompanying drawings.

In the accompanying drawings, an arrow F indicates a front direction of the illustrated structure. An arrow B indicates a rear direction of the illustrated structure. An arrow U indicates an upper direction of the illustrated structure. An arrow D indicates a lower direction of the illustrated structure. An arrow R indicates a right direction of the illustrated structure. An arrow L indicates a left direction of the illustrated structure.

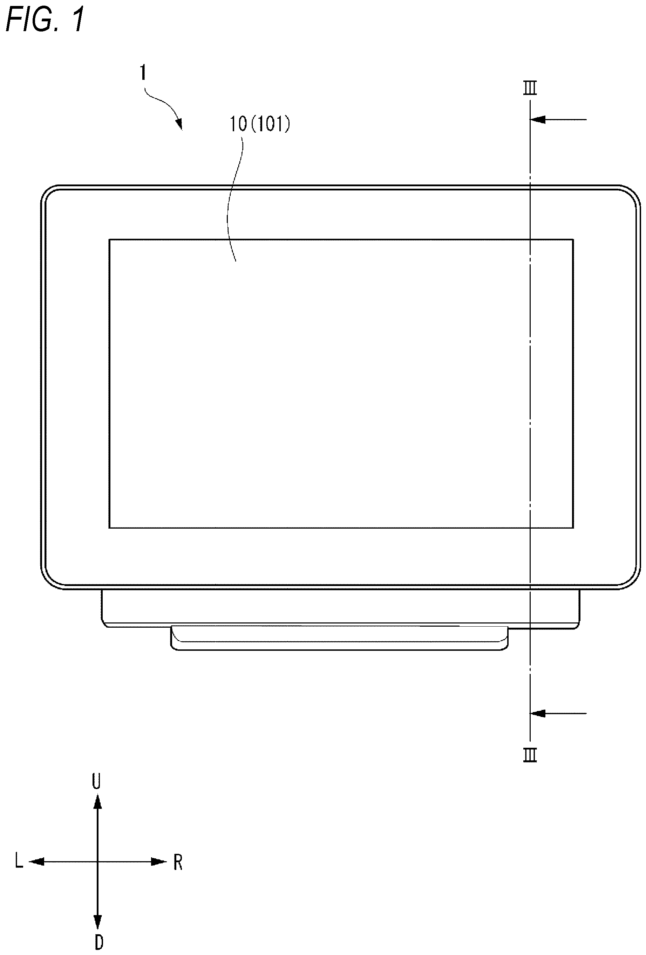

FIG. 1 illustrates an appearance of a vital monitor 1 according to an embodiment as viewed from the front. The vital monitor 1 is a device that acquires physiological information of a subject through a sensor attached to the subject. The vital monitor 1 is an example of a physiological information acquisition apparatus.

The vital monitor 1 can include a display device 10. The display device 10 is a device that displays an image including the acquired physiological information. In this example, the display device 10 can include a touch panel device 101. The touch panel device 101 is configured to detect a position to which a finger of a user approaches or touches, so as to receive an instruction from the user corresponding to the position.

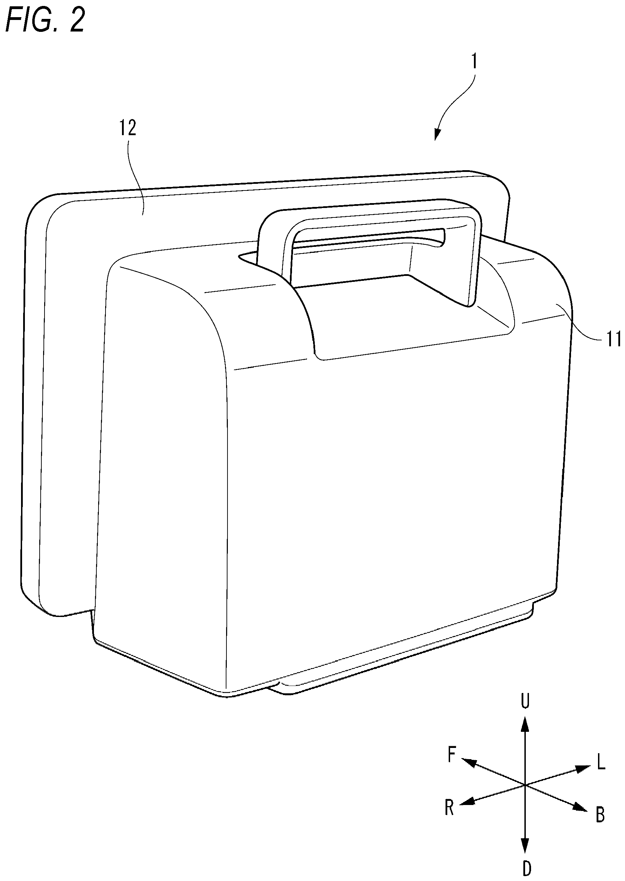

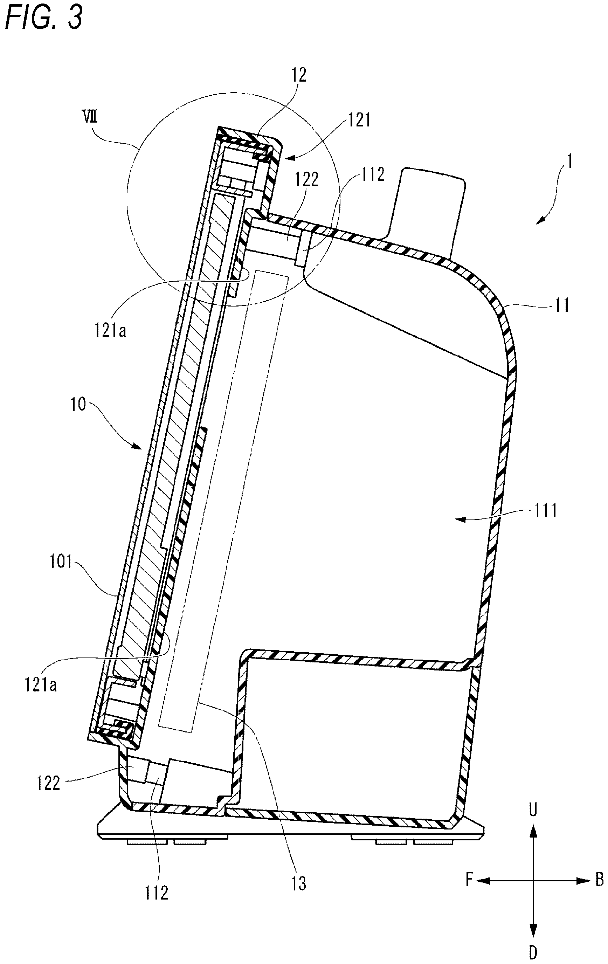

FIG. 2 illustrates an appearance of the vital monitor 1 as viewed from the right rear. FIG. 3 illustrates a cross section taken along a line III-III in FIG. 1 as viewed in a direction of arrows. The vital monitor 1 can include a first case 11, a second case 12, and a processing device 13.

As illustrated in FIG. 3, the first case 11 defines an internal space 111 that accommodates at least the processing device 13. The processing device 13 is a device that receives a detection signal corresponding to the physiological information of the subject from the sensor attached to the subject, and acquires the physiological information by performing appropriate processing on the signal.

The second case 12 supports the display device 10. The second case 12 can include a back wall 121. The back wall 121 can include a first portion 121a facing the internal space 111 defined by the first case 11.

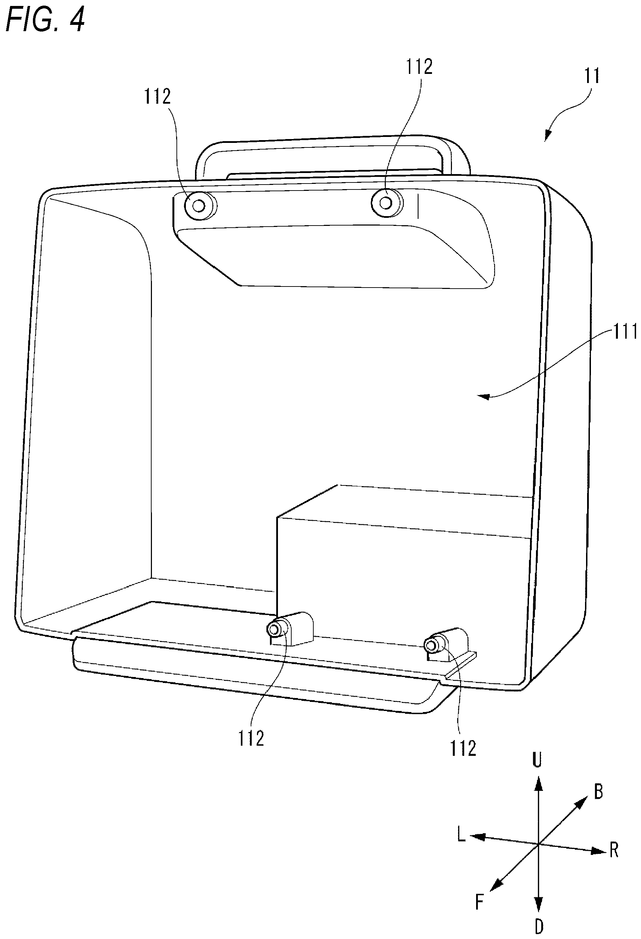

FIG. 4 illustrates an external appearance of the first case 11 as viewed from the right front. Illustration of component elements accommodated in the internal space 111 is omitted. The first case 11 can include coupling portions 112.

Meanwhile, as illustrated in FIG. 3, the second case 12 can include coupling portions 122. The coupling portions 122 are disposed at positions, where the coupling portions 122 can be coupled to the coupling portions 112 of the first case 11, on the first portion 121a of the back wall 121.

By coupling the coupling portions 112 of the first case 11 and the coupling portions 122 of the second case 12, a state in which the first case 11 and the second case 12 are coupled as illustrated in FIG. 2 is obtained. The coupling between the coupling portions 112 and the coupling portions 122 may be implemented by engagement or fitting, or may be implemented by screwing via a screw.

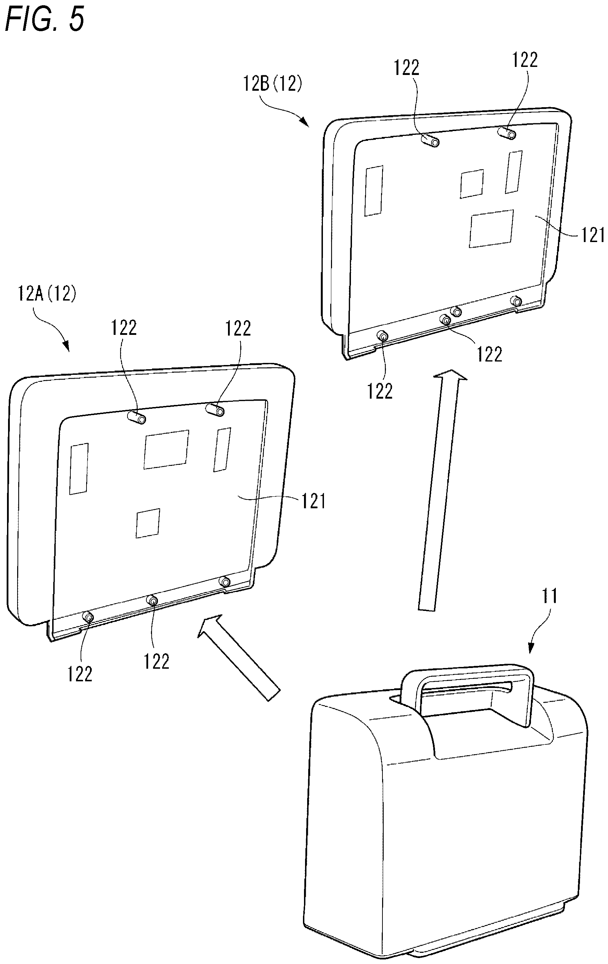

As illustrated in FIG. 5, the first case 11 according to the present embodiment can be commonly coupled to any of a plurality of types of second cases 12A and 12B. The second case 12A and the second case 12B are different from each other in size of the screen of the supported display device 10. That is, dimensions of the second case 12A and the second case 12B are also different.

On the other hand, the positions of the coupling portions 122 provided on the back wall 121 of the second case 12 are determined such that the coupling portions 122 can be coupled to the coupling portions 112 provided on the first case 11 regardless of the dimensions of the second case 12. Therefore, regardless of whether the second case 12A or the second case 12B is selected, the internal space 111, the coupling portions 112, the back wall 121, and the coupling portions 122 have a positional relationship illustrated in FIG. 4.

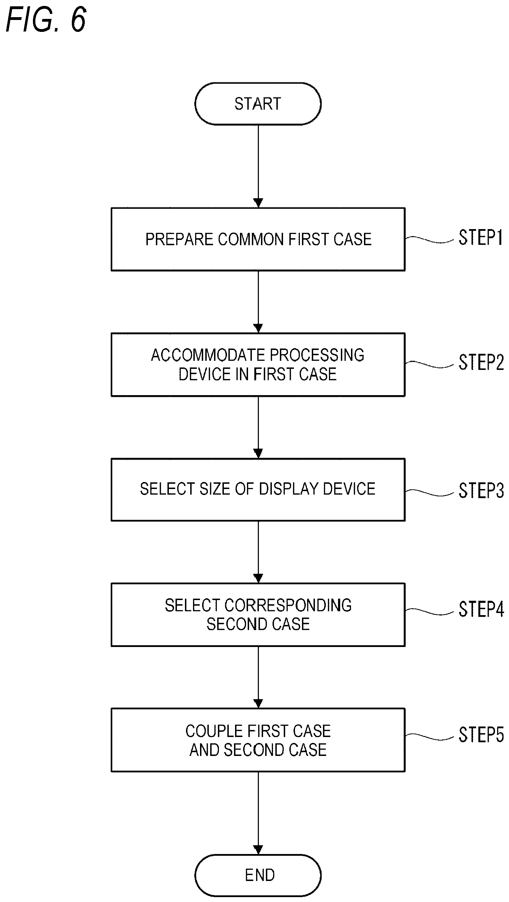

Therefore, according to a manufacturing method illustrated in FIG. 6, it is possible to obtain a plurality of types of vital monitors 1 in which screen sizes of the display devices 10 are different while using the first case 11 having a single specification.

Specifically, first, a common first case 11 is prepared (STEP 1). Subsequently, various component elements including the processing device 13 are accommodated in the internal space 111 of the first case 11 (STEP 2).

On the other hand, a plurality of types of display devices 10 including different sizes of screens on which the images showing the physiological information are displayed are prepared. Same or similarly, a plurality of types of second cases 12 each for supporting a corresponding type of display device 10 among the plurality of types of display devices 10 are prepared.

Subsequently, one type of display device 10 is selected from the plurality of types of display devices 10 according to a type of the vital monitor 1 to be manufactured (STEP 3). Same or similarly, one type of second case 12 corresponding to the selected type of display device 10 is selected from the plurality of types of second cases 12 (STEP 4).

Subsequently, the coupling portions 112 of the common first case 11 are coupled to the coupling portions 122 of the selected type of second case 12. Accordingly, the first case 11 and the first case 12 are coupled such that the first portion 121a of the back wall 121 of the selected type of second case 12 faces the internal space 111 of the common first case 11 (STEP 5).

According to the configuration described above, even when it is necessary to prepare a plurality of types of second cases 12 in order to support the display devices 10 having different screen sizes according to diversified needs, it is not necessary to prepare a plurality of types of first cases 11 having different specifications so as to correspond to the plurality of types of second cases 12. It is significant to eliminate the necessity of preparing a plurality of types of dies in order to manufacture the first case 11, for example, for efficiency. Therefore, an increase in the number of dies due to an increase in the number of types of the vital monitors 1 can be prevented.

As long as the processing device 13 is finally accommodated in the internal space 111 by coupling the first case 11 and the second case 12, the order of the processing illustrated in FIG. 6 may be appropriately changed. For example, instead of the step of accommodating the processing device 13 in the internal space 111 in STEP 2, the processing device 13 may be coupled to the first portion 121a of the back wall 121 of the second case 12 selected in STEP 4.

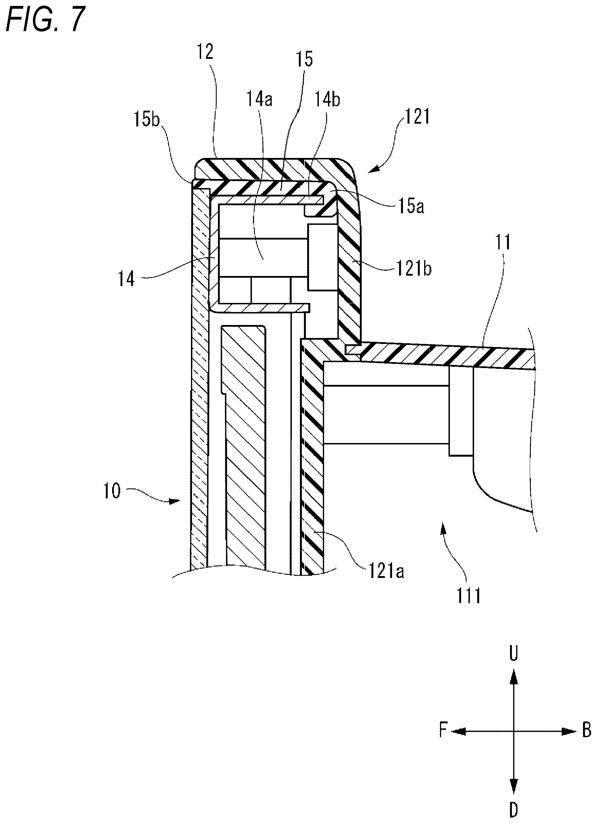

FIG. 7 illustrates an example of an enlarged view of an upper end portion of the display device 10 and an upper end portion of the second case 12 which are included in a region VII in FIG. 3. That is, the vital monitor 1 includes a frame member 14 and an elastic member 15.

The frame member 14 supports the display device 10. The frame member 14 can include a coupling portion 14a. The coupling portion 14a is coupled to the second case 12. Thus, the frame member 14 is supported by the second case 12. The frame member 14 can include a back portion 14b facing a second portion 121b of the back wall 121 of the second case 12. The second portion 121b of the back wall 121 is a portion that does not face the internal space 111 of the first case 11.

The elastic member 15 is formed as an integrally molded product of an elastic material such as rubber or elastomer. The elastic member 15 can include a first portion 15a. The first portion 15a of the elastic member 15 is disposed between the back portion 14b of the frame member 14 and the second portion 121b of the back wall 121.

Although not illustrated, the same structure is provided over an entire peripheral portion of the display device 10 and an entire peripheral portion of the second case 12.

According to such a configuration, the first portion 15a of the elastic member 15 can function as a buffer member, and can restrain not only external forces in an upper-lower direction and a left-right direction of the display device 10 but also an external force transmitted from the second case 12 side to the display device 10. In particular, in the present embodiment, when a plurality of types of second cases 12 having different dimensions are coupled to the common first case 11, the second portion 121b that does not face the internal space 111 is generated in the back wall 121. Since the first portion 15a of the elastic member 15 is disposed at a position facing the second portion 121b which is more likely to be exposed to an external force as compared with the first portion 121a facing the internal space 111, the effectiveness of the buffer function described above can be improved.

The elastic member 15 can include a second portion 15b. The first portion 15a and the second portion 15b are separate portions of the elastic member 15 which is an integrally molded product. The second portion 15b is disposed between the display device 10 and the second case 12. Although not illustrated, the same relationship is established over the entire peripheral portion of the display device 10 and the entire peripheral portion of the second case 12.

According to such a configuration, a sealing function of preventing liquid or dust from entering the vital monitor 1 from between the display device 10 and the second case 12 can be added to the elastic member 15 having the above-described buffer function.

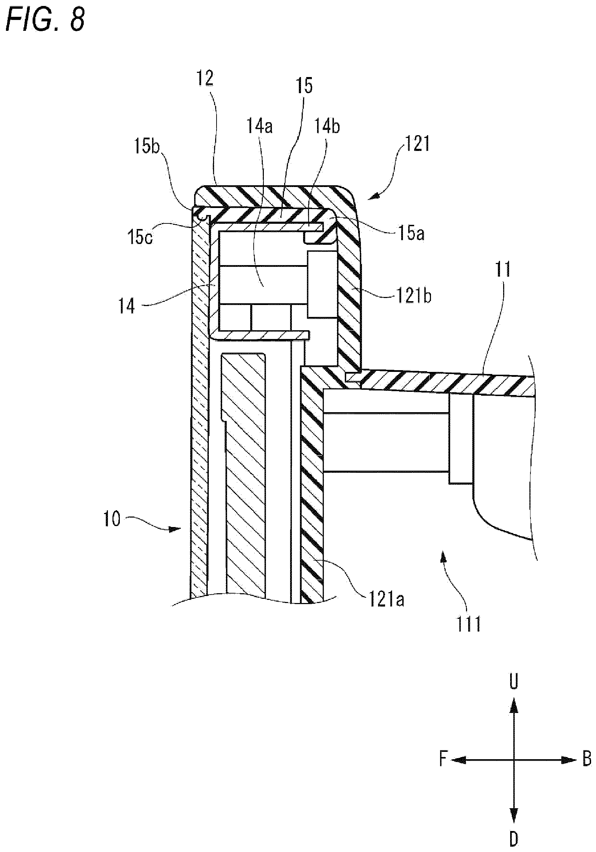

FIG. 8 illustrates another example of an enlarged view of an upper end portion of the display device 10 and an upper end portion of the second case 12 which are included in a region VII in FIG. 3. FIG. 8 differs from FIG. 7 in that the second portion 15b of the elastic member 15 has a bump portion 15c protruding from the second portion 15b. The bump portion 15c engages with a dent portion of the peripheral portion of the display device 10, and it makes it possible to fix the display device 10 stably. A shape of the bump portion 15c is not limited to a shape illustrated in FIG. 8. The number of bump portions 15c may be one or more.

The formation of the bump portion 15c reduces a contact area between the elastic member 15 and the peripheral portion of the display device 10 compared to a case where the bump portion 15c is not provided, and the interfacial pressure between the elastic member 15 and the display device 10 increases. Therefore, the interfacial pressure of the area where the elastic member 15 contacts with the display device 10 is increased, and it is possible to enhance the waterproof effect of the elastic member 15.

Moreover, the formation of the bump portion 15c cancels the decrease in interfacial pressure at the portion where the bump portion 15c contacts with the display device 10, even if there is a variation in dimensional tolerance of an opening of the manufactured second case 12. Therefore, contact between the bump portion 15c and the periphery of the display device 10 can be ensured, and it is possible to make the waterproof effect of the elastic member 15 higher compared to the case where the bump portion 15c is not provided.

The above embodiment is merely an example for facilitating the understanding of the presently disclosed subject matter. The configuration according to the above embodiment can be appropriately changed or improved without departing from the gist of the presently disclosed subject matter.

In FIG. 5, only two types of second cases 12 having different dimensions are illustrated. However, it is needless to say that three or more types of second cases 12 may be prepared according to the screen size of the display device 10 to be supported.

## Claims

1. A method for manufacturing a physiological information acquisition apparatus configured to acquire physiological information of a subject, the method comprising:
preparing a common first case that defines an internal space in which a processing device configured to perform processing of acquiring the physiological information is accommodated;
preparing a plurality of types of display devices including different sizes of screens for displaying an image including the physiological information;
preparing a plurality of types of second cases each for supporting a corresponding type of display device among the plurality of types of display devices;
selecting one type of display device from the plurality of types of display devices;
selecting, from the plurality of types of second cases, one type of second case corresponding to the selected one type of display device; and
coupling the first case to a coupling portion provided on a back wall of the second case, wherein the back wall faces the internal space.

2. A physiological information acquisition apparatus configured to acquire physiological information of a subject, comprising:
a processing device configured to perform processing of acquiring the physiological information;
a display device configured to display an image including the physiological information;
a first case that defines an internal space for accommodating the processing device; and
a second case configured to support the display device, wherein
the second case include
a back wall facing the internal space, and
a coupling portion provided on the back wall and coupled to the first case.

3. The physiological information acquisition apparatus according to claim 2, further comprising:
a frame member that supports the display device and is supported by the second case; and
an elastic member including a portion disposed between the back wall and a portion of the frame member facing the back wall.

4. The physiological information acquisition apparatus according to claim 3, wherein
the elastic member includes a portion disposed between the display device and the second case.
